# EUROPEAN PATENT APPLICATION

(11) **EP 1 647 255 A2**
(43) Date of publication of application: **19.04.2006**
(21) Application number: 05109522.2
(22) Date of filing: 13.10.2005
(51) Int. Cl.: A61J 1/00, A61M 5/178

(54) **Method for preparing sclerosing foams and medical device for implementing said method**

(30) Priority: 13.10.2004 IT VI20040246
(71) Applicant: Pentaferte Spa, 64012 Campli (IT)
(72) Inventor: ROMAGNOLI, Paolo Maria, 44100, FERRARA (IT)
(74) Representative: Bonini, Ercole

(57) **Abstract**

Method for preparing sclerosing foams consisting in the aspiration of sterile air (A) into a first syringe (2), the aspiration of a sclerosing liquid (L) into a second syringe (3), the connection of the two syringes (2, 3) so as to place the graduated cylinders (2a, 3a) of the syringes (2, 3) in communication with each other, and the mixing of the sterile air (A) with the sclerosing liquid (L) by means of the displacement of the plungers (6, 7) in the syringes (2, 3) to produce a sclerosing foam. Said mixing of the sterile air (A) with the sclerosing liquid (L) is achieved by the displacement of the plungers (6, 7) in the syringes (2, 3) in the longitudinal direction (Z) of the coupling of the syringes (2, 3), coinciding with the axis of displacement of each one of the plungers (6, 7).

## Description

The present invention relates to a method for preparing sclerosing foams and a medical device for implementing said method.

Known systems currently used for the treatment of varices, i.e. enlarged, dilated and tortuous lymph or blood vessels (e.g. veins) consist in preparing a sclerosing mousse or foam by mixing sterile air with a detergent type of sclerosing liquid, generally tetradecylsulfate (TDS) or polydocanol (POL), in suitable proportions depending on the required density of the resulting foam.

This medical practice, called "sclerotherapy", is particularly effective in the treatment of medium- and large-caliber varices (e.g. truncular, recurrent, due to perforation or to saphenous or collateral branch incontinence, to name just a few).

The sclerosing foam is inserted in the varix by a specialist physician, where it triggers an inflammatory reaction of the vein and the subsequent formation of a thrombus.

This leads to the occlusion of the diseased vein, a phenomenon better known as "sclerosis" - hence the name of sclerosing foam, and prevents the blood flow in the vein.

The consequent atrophy of the diseased vein is the first step in the definitive healing of the varix.

Among the methods currently used to produce sclerosing mousses, the most widespread is the so-called "Tessari method", from the name of the specialist physician who introduced it at the end of the Nineties.

This method consists in preparing a sclerosing foam with the aid of a pair of syringes fitted with an end portion called a "luer lock", that is to say with a taper angle of 6%.

The dimensions of the two syringes generally differ and they are connected together by means of a connector valve ensuring perfect air tightness, which is essentially a three-way valve with a through hole.

The procedure used to obtain a sclerosing mousse consists in connecting the two syringes to corresponding connector valve outlets, then attaching a sterile needle to the free connector valve outlet opposite the larger syringe.

With the syringes arranged in said position, a given quantity of sclerosing liquid, generally 0.5 ml, is aspirated into the smaller syringe directly from a sealed ampoule or bottle.

The user then rotates the connector valve to place the inner cylinders of the two syringes in communication with each other and, using both hands and beginning with the larger-diameter syringe, transfers first the air and then the sclerosing liquid from one syringe to the other, thereby mixing the emulsion.

Rapidly repeating these steps a suitable number of times gives rise to a compact sclerosing foam of excellent quality, i.e. with the density needed to adhere to the wall of the diseased vein, occluding the vessel without interfering with the blood flow.

Using the above-described method, approximately 20 passages of the mixture from one syringe to the other are needed to ensure a good quality sclerosing foam.

Having completed this process, the physician-user detaches the smaller syringe containing the required quantity of sclerosing foam from the three-way valve and attaches a sterile needle to said syringe in order to inject said sclerosing foam into the varix being treated.

It is essential for the sclerosing foam to be injected into the diseased vein as soon as it is ready, because this is when it possesses the right density characteristics for ensuring an effective treatment of the varix.

The particular feature of the Tessari method, moreover, lies in that it can produce stable and compact sclerosing foams even using plastic syringes, thus avoiding the problems relating to the use of glass syringes.

It is also a very straightforward, safe and extremely practical method for making sclerosing foams.

Even the Tessari method nonetheless has some drawbacks.

Its main drawback derives from the fact that the syringes are arranged substantially perpendicular to each other during the preparation of the sclerosing mousse, making their handling somewhat complicated and unnatural.

This is due to the type of connector valve used to join the two syringes, which is a three-way valve, as mentioned previously.

In fact, the user has to change the orientation of the valve from an initial position, which places the ampoule in communication with the smaller syringe to enable the aspiration of the sclerosing liquid, to a final position that places the two syringes in communication with each other, to enable the liquid to be mixed with the sterile air. This makes the procedure for implementing the Tessari method rather laborious and inevitably adds to the time it takes to obtain the sclerosing foam.

The present invention intends to overcome the above-stated drawbacks.

In particular, the main object of the present invention is to simplify the procedures for preparing sclerosing foams for treating varices, by comparison with the methods of the known state of the art.

Another object of the invention is to enable a more rapid preparation of sclerosing foams than is achievable with the known state of the art.

The above-stated objects are achieved by a method for preparing sclerosing foams that, in accordance with the contents of the main claim, consists of the following steps:
- the aspiration of sterile air into a first syringe;
- the aspiration of sclerosing liquid into a second syringe;
- the connection of said two syringes to place the graduated cylinders of said syringes in communication with each other;
- the mixing of said sterile air with said sclerosing liquid by means of the actuation of the plungers of said syringes to obtain said sclerosing foam,
characterized in that said procedure for mixing said sterile air with said sclerosing liquid involves the displacement of said plungers inside said syringes in the longitudinal direction of the coupling between said syringes, coinciding with the axis of displacement of each of said plungers.

The method of the invention is implemented using a medical device for preparing sclerosing foams (also covered by the present patent) comprising:
- a first syringe with a first hollow cylinder suitable for containing sterile air, wherein a first plunger slides, attached to the end of a first rod that can be actuated by the user;
- a second syringe with a second hollow cylinder suitable for containing a sclerosing liquid, wherein a second plunger slides, attached to the end of a second rod that can be actuated by the user;
- connection means between said syringes, suitable for placing the graduated cylinders of said syringes in communication with each other,
characterized in that said connection means are in the form of a connector valve suitable for removably coupling said syringes with each other in a longitudinal direction coinciding with the axis of displacement of each of said plungers.

Advantageously, the invention enables a more straightforward and practical preparation of sclerosing foams for the treatment of varices than is achievable with the known state of the art.

The invention also advantageously enables a reduction in the time it takes to prepare good quality sclerosing foams with respect to the known state of the art.

Further characteristics and particular features of the invention will be better clarified in the description of a preferred embodiment of the invention provided herein as a non-restrictive example with reference to the attached drawings, wherein:
- Figure 1 is an axonometric view of the medical device subject of the invention;
- Figure 2 is an exploded axonometric view of the device shown in Figure 1, together with other components thereof;
- Figure 3 is a cross section of an enlarged detail of the device shown in Figure 1.

The method of the invention is described with reference to a medical device for preparing sclerosing foams illustrated in the attached drawings, wherein it is indicated as a whole by the numeral **1.**

The method consists in the following steps:
- the aspiration of sterile air **A** into a first syringe, indicated as a whole by the numeral **2;**
- the aspiration of a sclerosing liquid **L** into a second syringe, indicated as a whole by the numeral **3;**
- the connection of the two syringes **2, 3** in order to place the graduated cylinders **2a, 3a** of the syringes **2, 3** in communication with each other;
- the mixing of the sterile air **A** with the sclerosing liquid **L** by means of the displacement of the plungers **6, 7** in the syringes **2, 3** to obtain a sclerosing foam.

According to the invention, the procedure for mixing the sterile air **A** with the sclerosing liquid **L** involves displacing the plungers **6, 7** in the syringes **2, 3** in the longitudinal direction **Z** in which the syringes **2, 3** are coupled, which coincides with the axis of displacement of the two plungers **6, 7.**

The displacement of the plungers **6, 7** preferably, but not necessarily, takes place at different times and in opposite directions, enabling the rapid preparation of an excellent quality sclerosing foam.

Clearly, in other variants of the invention, the displacement of the plungers in the aforesaid syringes may take place in the same direction.

In other variants of the invention, said displacement of the plungers in the syringes may occur simultaneously, in the same direction or in opposite directions.

The method of the invention thus clearly achieves the objects of quickly and easily preparing sclerosing foams of a given density.

After connecting the two syringes together, the user immediately begins to produce the sclerosing foam, handling the two syringes (aligned one opposite the other) with ease, without the need to perform the additional procedures, such as changing the position of the connector valve, required by the Tessari method.

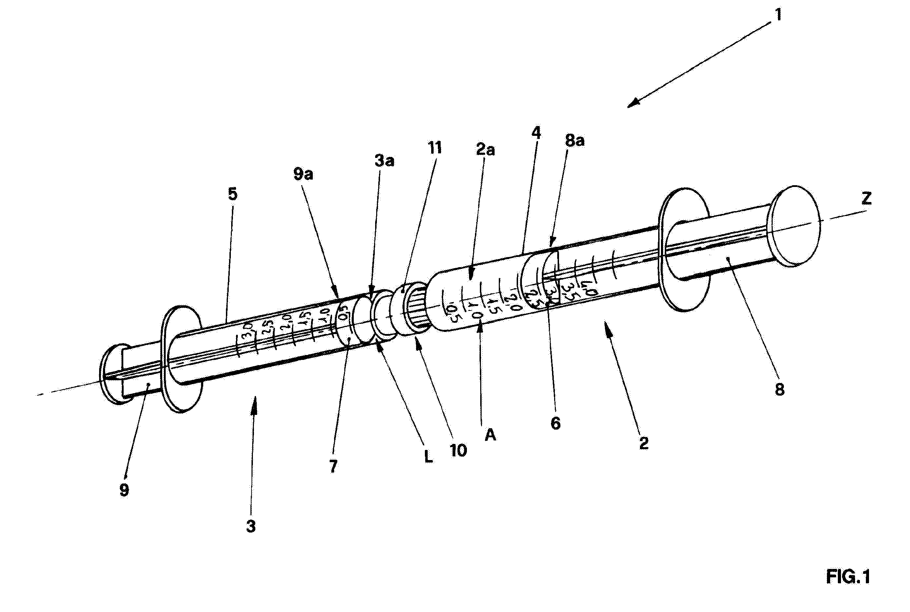

The medical device **1** for implementing the above-described method is illustrated, configured for use, in Figure 1.

It consists of a first syringe **2** complete with a first hollow cylinder **4,** for containing sterile air **A,** wherein a first plunger **6** attached to the end **8a** of a first rod **8** is slidingly engaged, said rod being actuated by the user.

The medical device **1** also consists of a second syringe **3** with a second hollow cylinder **5,** for containing a sclerosing liquid **L,** such as tetradecylsulfate or polydocanol, wherein a second plunger **7** attached to the end **9a** of a second rod **9** is slidingly engaged, said rod being actuated by the user.

The medical device **1** also comprises means, indicated as a whole by the numeral **10,** for connecting the syringes **2, 3,** which place the graduated cylinders **2a, 3a** of the syringes **2, 3** in communication with each other.

According to the invention, the connection means **10** consist of a connector valve **11** that enables the syringes **2, 3** to be removably coupled to each other in the longitudinal direction **Z** coinciding with the axis of displacement of both the plungers **6, 7.**

As illustrated in all the attached figures, the first hollow cylinder **4** of the first syringe **2** is preferably, but not necessarily, larger in diameter than the second hollow cylinder **5** of the second syringe **3.**

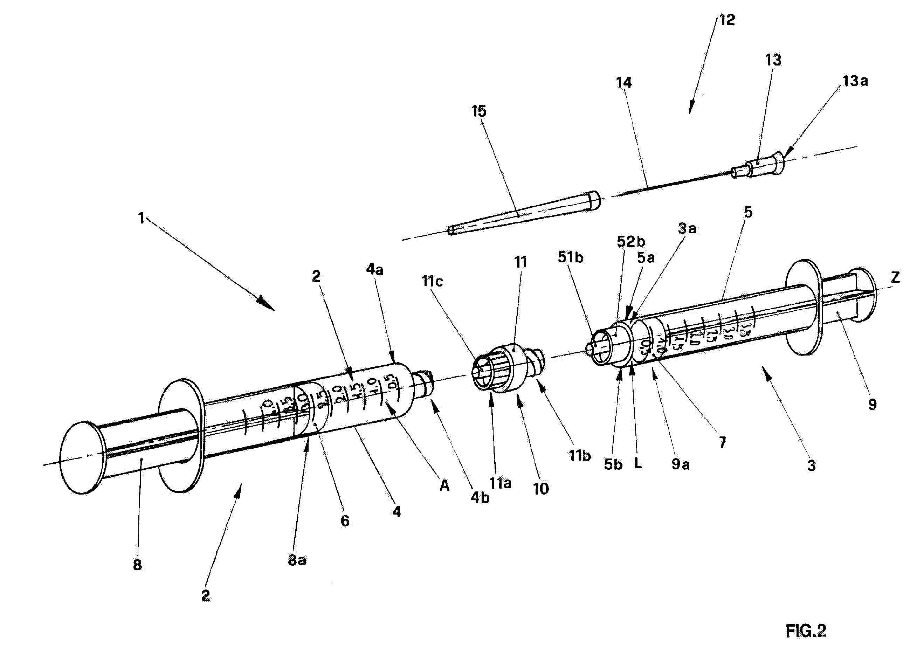

The connector valve **11,** better illustrated in the exploded view shown in Figure 2, is of known type and, to be precise, it is of the so-called "luer lock" type, as mentioned earlier, i.e. with a taper angle of 6%, forming the object of the US patent 5,775,671 and currently used in other applications for various purposes, such as infusions.

As shown in Figure 2, the first hollow cylinder **4** and the second hollow cylinder **5** each have a tubular fitting **4b, 5b** at one end **4a, 5a** for coupling coaxially on opposite sides of the connector valve **11** to achieve an airtight connection.

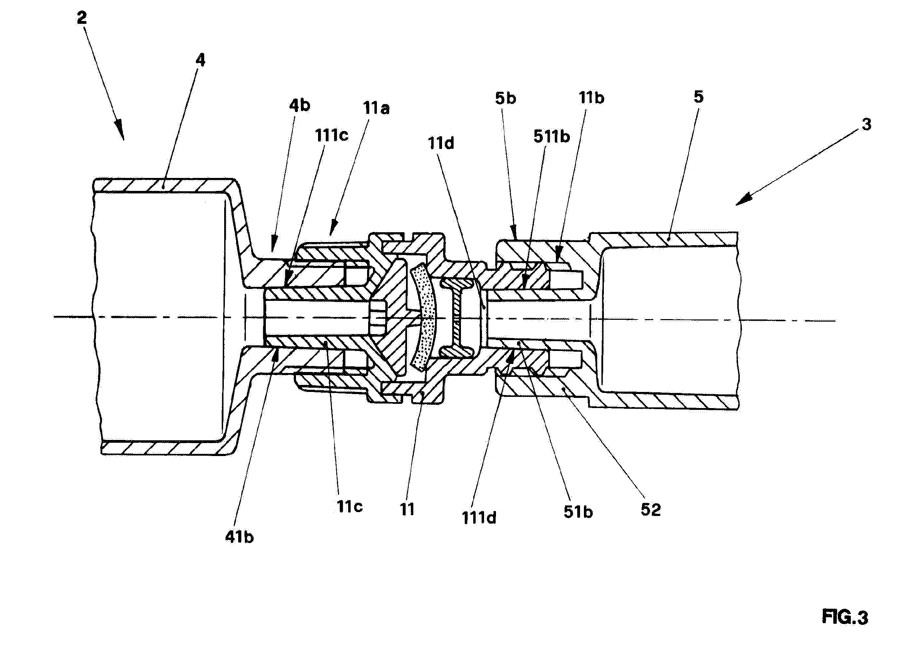

To be more precise, as shown in Figure 2 and in greater detail in Figure 3, the tubular fitting **4b** on the first hollow cylinder **4** is provided with a female "luer lock" taper angle **41b** for coupling with a tubular projection **11c** that in turn is provided with a male "luer lock" taper angle **111c** situated at one end **11a** of the connector valve **11.**

The tubular fitting **5b** on the second hollow cylinder **5,** on the other hand, consists of a central part **51b** with a male "luer lock" taper angle **511b** for engaging in a cavity **11d** with a female "luer lock" taper angle **111d** at the other end **11b** of the connector valve **11.**

Figures 2, 3 also show that the coupling between the connector valve **11** and the tubular fitting **4b, 5b** on the first hollow cylinder **4** and on the second hollow cylinder **5,** respectively, is of the threaded type.

The male thread is cut on the tubular fitting **4b** on the first hollow cylinder **4** and on the second end **11b** of the connector valve **11,** while the female thread is cut on the inside of a peripheral wall **52b,** surrounding the central part **51 b** of the tubular fitting **5b** on the second hollow cylinder **5** and on the first end **11a** of the connector valve **11.**

According to the preferred embodiment of the invention described herein, and with reference to Figure 2, the medical device **1** also comprises a needle, generically indicated by the numeral **12,** that is used either to collect the sclerosing liquid L from a container, such as an ampoule (not shown), during the initial stages of the process for preparing the sclerosing foam, or to inject the sclerosing foam that has just been prepared by means of said process into the lymph or blood vessel being treated.

Note that the needle **12** consists of a shaped holder **13** with a female "luer lock" taper angle **13a** that is coupled to the central part **51 b** of the tubular fitting **5b** on the second hollow cylinder **5,** when the sclerosing liquid **L** has to be taken from the ampoule.

The needle **12** also comprises a cannula **14,** attached to the holder **13,** which is protected by a cover **15** with a substantially truncated cone shape when the needle **12** is not in use.

In practice, the user (e.g. the specialist physician) arranges the first syringe **2** with its plunger **6** partially withdrawn, so that the first hollow cylinder **4** contains sterile air **A,** the quantity of which varies depending on room temperature.

Then the user draws the sclerosing liquid **L** from the ampoule into the second syringe **3** with the aid of a needle of the type indicated by the numeral **12,** coupling its shaped holder **13** to the central part **51 b** of the second hollow cylinder **5.**

To ensure the sclerosing foam has the required density, the first hollow cylinder **4** preferably contains 2.5 ml of sterile air **A,** while 0.5 ml of sclerosing liquid **L** (e.g. tetradecylsulfate) are drawn into the second hollow cylinder **5.**

After detaching the needle **12** from the second syringe **3,** the user axially connects the two syringes **2, 3** by inserting the connector valve **11** between them, achieving an airtight coupling by screwing the tubular fitting **4b, 5b** on the first hollow cylinder **4** and on the second hollow cylinder **5,** respectively, on opposite sides of the connector valve **11.**

The user then begins to prepare the sclerosing foam by simultaneously gripping the first rod **8** and the second rod **9** of the corresponding syringes **2, 3,** and transferring the sterile air **A** and the sclerosing liquid **L** from one syringe to the other.

The sterile air **A** and the sclerosing liquid **L** at the beginning, as well as the emulsion produced by mixing them together at a later moment, can flow in both directions through the connector valve **11,** as explained in the US patent 5,775,671.

The user withdraws the first rod **8** and the second rod **9,** one after the other and in opposite directions, and then pushes them inwards again, thereby bringing the plungers **6, 7** to bear on the emulsion progressively being prepared to produce the sclerosing foam.

Repeating the above-described steps several times quickly and easily gives rise to sclerosing foams of suitable density.

After completing these steps, the user detaches the connector valve **11** from the second syringe **3** containing the required quantity of sclerosing foam and attaches a new sterile needle, of the type indicated by the numeral **12,** to said syringe, with which the sclerosing foam is injected into the site to treat.

The description of the medical device **1** more effectively and clearly demonstrates the advantages offered by the present invention, deriving particularly from the use of a connector valve of the type indicated by the numeral **11,** which enables the rapid preparation of the sclerosing mousse without the laborious procedure involved in the equivalent methods of the known state of the art.

The solution relies on the fact that there are two syringes **2, 3** of the type described, which are easily coupled with a needle featuring a corresponding "luer lock" taper angle to be used to draw the sclerosing liquid **L** into the second syringe 3 before attaching it to the connector valve **11.**

On the strength of the above description, it is clear that the invention achieves all the objects set and offers all the advantages previously stated.

Upon implementation, changes may be made to the medical device subject of the invention, consisting for instance in a different coupling between the two syringes and the connection means along the axis of displacement of the plungers of said syringes.

Moreover, there may be practical variants of the method of the invention, consisting for instance in steps for drawing the sclerosing liquid into a different syringe from those described herein, without this affecting the coverage afforded by the present patent.

Moreover, the syringes used may be made of any material, even though the use of plastic is considered preferable.

Should they come within the scope of the following claims, any variants described or mentioned, but not illustrated in the attached drawings, shall in any case be deemed covered by the present patent.

## Claims

1. Method for preparing sclerosing foams consisting in the following steps:
- the aspiration of sterile air (A) into a first syringe (2);
- the aspiration of a sclerosing liquid (L) into a second syringe (3);
- the connection between said syringes (2, 3) to place the graduated cylinders (2a, 3a) of said syringes (2, 3) in communication with each other;
- the mixing of said sterile air (A) with said sclerosing liquid (L) by means of the actuation of the plungers (6, 7) of said syringes (2, 3) to obtain said sclerosing foam,
**characterized in that** said procedure for mixing of said sterile air (A) with said sclerosing liquid (L) involves the displacement of said plungers (6, 7) in said syringes (2, 3) in the longitudinal direction (Z) in which said syringes (2, 3) are coupled, which coincides with the axis of displacement of said plungers (6, 7).

2. Method according to claim 1), **characterized in that** said displacement of said plungers (6, 7) takes place in opposite directions.

3. Method according to claim 1), **characterized in that** said displacement of said plungers takes place in the same direction.

4. Method according to claim 1), **characterized in that** said displacement of said plungers (6, 7) takes place simultaneously.

5. Method according to claim 1), **characterized in that** said displacement of said plungers (6, 7) takes place at different times.

6. Medical device (1) for preparing sclerosing foams, comprising:
- a first syringe (2) with a first hollow cylinder (4), suitable for containing sterile air (A), wherein a first plunger (6) attached to the end (8a) of a first rod (8) is slidingly engaged, said rod being actuated by the user;
- a second syringe (3) with a second hollow cylinder (5), suitable for containing a sclerosing liquid (L), wherein a second plunger (7) attached to the end (9a) of a second rod (9) is slidingly engaged, said rod being actuated by the user;
- means (10) for connecting said syringes (2, 3) so as to place the graduated cylinders (2a, 3a) of said syringes (2, 3) in communication with each other,
**characterized in that** said connection means (10) are in the form of a connector valve (11) suitable for removably coupling said syringes (2, 3) together in the longitudinal direction (Z) coinciding with the axis of displacement of said plungers (6, 7).

7. Medical device (1) according to claim 6), **characterized in that** said first hollow cylinder (4) and said second hollow cylinder (5) each have a tubular fitting (4b, 5b) at one end (4a, 5a) for coupling coaxially on opposite sides of said connector valve (11) to create an airtight seal.

8. Medical device (1) according to claim 7), **characterized in that** said tubular fitting (4b) on said first hollow cylinder (4) is provided with a female "luer lock" taper angle (41 b) for coupling with a tubular projection (11c) provided with a male "luer lock" taper angle (111c) on a first end (11a) of said connector valve (11).

9. Medical device (1) according to claim 7), **characterized in that** said tubular fitting (5b) on said second hollow cylinder (5) comprises a central part (51 b) with a male "luer lock" taper angle (511 b) for engaging in a cavity (11d) with a female "luer lock" taper angle (111d), provided on a second end (11b) of said connector valve (11).

10. Medical device (1) according to claim 9), **characterized in that** it comprises a needle (12) to be used to take said sclerosing liquid (L) from a container or to inject said sclerosing foam into the lymph or blood vessel being treated, said needle (12) consisting of:
- a shaped holder (13) with a female "luer lock" taper angle (13a) for coupling with said central part (51 b) of said tubular fitting (5b) on said second hollow cylinder (5);
- a cannula (14) attached to said shaped holder (13), protected by a cover (15) when said needle (12) is not in use.

11. Medical device (1) according to claim 9), **characterized in that** said coupling is of the threaded type.

12. Medical device (1) according to claim 11), **characterized in that** the male thread is cut on said tubular fitting (4b) of said first hollow cylinder (4) and on the second end (11 b) of said connector valve (11).

13. Medical device (1) according to claim 11), **characterized in that** the female thread is cut on a peripheral wall (52b) surrounding said central part (51 b) of said tubular fitting (5b) of said second hollow cylinder (5) and on the first end (11 a) of said connector valve (11).

14. Medical device (1) according to claim 6), **characterized in that** said first hollow cylinder (4) has a larger diameter than said second hollow cylinder (5).
